# EUROPEAN PATENT APPLICATION

(11) **EP 1 683 855 A1**
(43) Date of publication of application: **26.07.2006**
(21) Application number: 05255482.1
(22) Date of filing: 07.09.2005
(51) Int. Cl.: C12M 3/00, C12N 15/89

(54) **Cell capturing apparatus and method of capturing cell**

(30) Priority: 21.01.2005 JP 2005014588
(71) Applicant: FUJITSU LIMITED, Kawasaki-shi, Kanagawa 211-8588 (JP)
(72) Inventor: Harada, Tohru, c/o Fujitsu Limited, Kawasaki-shi, Kanagawa 211-8588 (JP); Ando, Moritoshi, c/o Fujitsu Limited, Kawasaki-shi, Kanagawa 211-8588 (JP); Mishima, Kazuhisa, c/o Fujitsu Limited, Kawasaki-shi, Kanagawa 211-8588 (JP); Sakai, Satoru, c/o Fujitsu Limited, Kawasaki-shi, Kanagawa 211-8588 (JP); Youoku, Sachihiro, c/o Fujitsu Limited, Kawasaki-shi, Kanagawa 211-8588 (JP)
(74) Representative: Fenlon, Christine Lesley

(57) **Abstract**

A path (17) defines an opening smaller than a cell in a cell capturing apparatus (11). A negative pressure generating device (22) is connected to the path so as to generate negative pressure within the path. A negative pressure controlling device (23) is connected to the path so as to adjust the negative pressure within the path. A cell is caught at the opening of the path based on the negative pressure. The negative pressure controlling device serves to maintain the negative pressure constant within the path. Even if the negative pressure changes within the path based on drop or rise in the temperature within the path, for example, the negative pressure can be adjusted at a level suitable for the caught cell with the assistance of the negative pressure controlling device. The cell can reliably be kept immobilized at the opening of the path.

## Description

The present invention relates to a cell capturing apparatus designed to capture cells in a solution. The cell capturing apparatus may be utilized in a microinjection apparatus designed to inject a predetermined solution into cells under a microscope, for example.

A microinjection apparatus is disclosed in Japanese Patent Application Publication 62-270197, for example. The microinjection apparatus includes a capturing board capable of catching cells. Paths are defined in the capturing board. When negative pressure is generated within the paths with the assistance of a suction pump, the cells dispersed in the solution are caught at the openings of the paths. Here, medicine is injected into the cells by means of an injector, for example.

The negative pressure within the paths inevitably changes due to the pulsation of the suction pump and/or a change in environmental temperature. If the negative pressure within the paths increases, for example, cells are sucked down into the paths. On the other hand, if the negative pressure decreases, cells are allowed to move away from the openings of the paths. As a result, cells cannot reliably be immobilized at the openings of the paths.

It is accordingly desirable to provide a cell capturing apparatus contributing to a reliable capture of cells under any circumstances. It is also desirable to provide a method of capturing cells contributing to a reliable capture of cells under any circumstances.

According to one embodiment of a first aspect of the present invention, there is provided a cell capturing apparatus comprising: a path defining an opening smaller than a cell; a negative pressure generating device connected to the path, said negative pressure generating device generating negative pressure within the path; and a negative pressure controlling device connected to the path, said negative pressure controlling device adjusting the negative pressure within the path.

The cell capturing apparatus allows the negative pressure generating device to generate negative pressure within the path. A cell is thus caught at the opening of the path based on the negative pressure. The negative pressure controlling device serves to maintain the negative pressure constant within the path. Even if the negative pressure changes within the path based on drop or rise in the temperature within the path, for example, the negative pressure can be adj usted at a level suitable for the caught cell with the assistance of the negative pressure controlling device. The cell can reliably be kept immobilized at the opening of the path.

The cell capturing apparatus may further comprise : a liquid reservoir defining a closed space; a first piping member connected to the path, said first piping member having an opening within the liquid reservoir at a location spaced from the bottom of the liquid reservoir; and a second piping member connected to the negative pressure generating device, said second piping member having an opening within the liquid reservoir at a location spaced from the bottom of the liquid reservoir.

The cell capturing apparatus allows dispersion of cells in a solution such as a culture medium, for example. When negative pressure is generated within the path, the solution drops toward the bottom of the liquid reservoir through the first piping member based on the negative pressure. The solution is kept on the bottom of the liquid reservoir. As long as the opening of the second piping member is sufficiently spaced from the surface of the solution, the second piping member is reliably prevented from receiving the inflow of the solution. Only air is allowed to flow into the second piping member from the first piping member. Accordingly, the negative pressure generating device is reliably prevented from suffering from receiving the inflow of the solution. If the solution flows into the negative pressure generating device, the negative pressure generating device is supposed to break down based on the inflow of liquid such as the solution. Avoidance of the inflow of the solution in this manner greatly contributes to a reliably continuous operation of the negative pressure generating device. The negative pressure can accurately be adjusted.

The cell capturing apparatus may be utilized in a microinjection apparatus. One embodiment of a second aspect of the present invention provides a microinjection apparatus comprising: a path defining an opening smaller than a cell; a negative pressure generating device connected to the path, said negative pressure generating device generating negative pressure within the path; a negative pressure controlling device connected to the path, said negative pressure controlling device adjusting the negative pressure within the path; and an injector injecting a predetermined injectant into the cell. The microinjection apparatus enables establishment of a stable negative pressure suitable for the cell in the same manner as described above. The cell can reliably be kept immobilized at the opening of the path. The injectant can be injected into the cell in an efficient manner. The injection can be achieved in a shorter time.

According to one embodiment of a third aspect of the present invention, there is provided a method of capturing a cell, comprising: filling a path with solution, said path defining an opening smaller than the cell; supplying the cell to the opening of the path; and generating a predetermined negative pressure within the path so as to immobilize the cell at the opening of the path.

The method allows the path to be filled up with the solution prior to the supply of the cell. The solution is thus reliably prevented from suffering from mixture of air within the path. Generation of the surface tension can be avoided at the boundary between air and the solution. This contributes to an accurate control on the negative pressure.

The above and other features and advantages of the present invention will become apparent from the following description of the preferred embodiments by way of example in conjunction with the accompanying drawings, wherein:
Fig. 1 is a schematic view illustrating the structure of a microinjection apparatus including a cell capturing apparatus according to a first embodiment of the present invention;
Fig. 2 is a partial plan view of the microinjection apparatus for schematically illustrating the structure of a silicon chip according to an embodiment of the invention;
Fig. 3 is a partial sectional view taken along the line 3-3 in Fig. 2;
Fig. 4 is a partial sectional view of the silicon chip, corresponding to Fig. 3, for schematically illustrating the capture of cells;
Fig. 5 is a schematic view illustrating the structure of a microinjection apparatus including a cell capturing apparatus according to a second embodiment of the present invention; and
Fig. 6 is a schematic view illustrating the structure of a syringe according to an embodiment of the present invention.

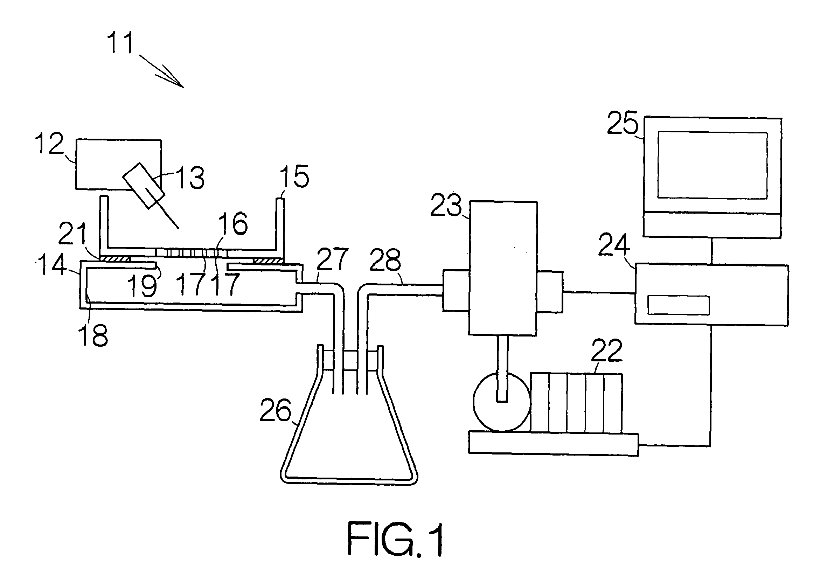

Fig. 1 schematically illustrates the structure of a microinjection apparatus 11 including a specific example of a cell capturing apparatus according to a first embodiment of the present invention. The microinjection apparatus 11 includes an injection mechanism 12. The injection mechanism 12 includes an injector or capillary 13. An injectant such as liquid or the like is injected into cells from the tip end of the capillary 13. The injection mechanism 12 is opposed to a work stage 14. The capillary 13 is allowed to move relative to the work stage 14.

A removable Petri dish 15 is mounted on the horizontal plane defined on the work stage 14. A silicon chip 16 is fixed in the Petri dish 15. Paths 17, 17, ... are formed in the silicon chip 16. The paths 17 penetrate through the silicon chip 16 across the thickness. The thickness of the silicon chip 16 is set at 10µm approximately, for example. The silicon chip 16 may have a rectangular periphery, for example. The size of the silicon chip 16 may be set at 10mm by 10mm approximately, for example.

The work stage 14 includes a pressure chamber 18. An opening 19 is formed in the pressure chamber 18. The opening 19 is opened at the horizontal plane. A seal member 21 is located around the opening 19 on the horizontal plane of the work stage 14. The seal member 21 endlessly surrounds the opening 19 at the horizontal plane. The seal member 21 receives the flat bottom of the Petri dish 15. Therefore, the pressure in the pressure chamber 18 influences the pressure in the paths 17 defined in the silicon chip 16.

A negative pressure generating device or vacuum pump 22 is connected to the pressure chamber 18. The vacuum pump 22 is capable of sucking air from the pressure chamber 18 with a predetermined suction. The vacuum pump 22 is in this manner capable of generating negative pressure in the pressure chamber 18. A negative pressure controlling device or electropneumatic regulator 23 is interposed between the pressure chamber 18 and the vacuum pump 22. The electropneumatic regulator 23 is designed to keep the negative pressure constant within the pressure chamber 18. The negative pressure can be set at a predetermined level based on the value of voltage supplied to the electropneumatic regulator 23.

A digital/analog converter, DAC, 24 is connected to the electropneumatic regulator 23. The digital/analog converter 24 supplies electricity or voltage to the electropneumatic regulator 23. A computer 25 is connected to the digital/analog converter 24. A digital signal supplied from the computer 25 is utilized to determine the value of voltage supplied to the electropneumatic regulator 23. For example, the computer 25 holds the initial values of the negative pressure suitable for each kind of cell. The computer 25 derives the value of voltage for the electropneumatic regulator 23 so as to realize the initial value of the negative pressure. The computer 25 transmits to the digital/analog converter 24 a digital signal specifying the value of voltage.

A liquid reservoir 26 is interposed between the pressure chamber 18 and the electropneumatic regulator 23. The liquid reservoir 26 defines a closed space. The liquid reservoir 26 is designed to hold a culture medium or suspension within the closed space. A first piping member or tube 27 is utilized to connect the liquid reservoir 26 to the pressure chamber 18. The first tube 27 has an opening within the liquid reservoir at a location spaced from the bottom of the liquid reservoir 26. In this case, the opening of the first tube 27 is directed downward in the direction of the gravity. The opening of the first tube 27 is thus set spaced from the upper surface of the culture medium or suspension stored in the liquid reservoir 26.

A second piping member or tube 28 is utilized to connect the liquid reservoir 26 to the electropneumatic regulator 23. The second tube 28 has an opening within the liquid reservoir 26 at a position spaced from the bottom of the liquid reservoir 26. In this case, the opening of the second tube 28 is directed downward in the direction of the gravity in the same manner as the first tube 27. The opening of the second tube 28 is thus spaced from the upper surface of the culture medium or suspension stored within the liquid reservoir 26. The liquid reservoir 26 in this manner serves as a so-called trap.

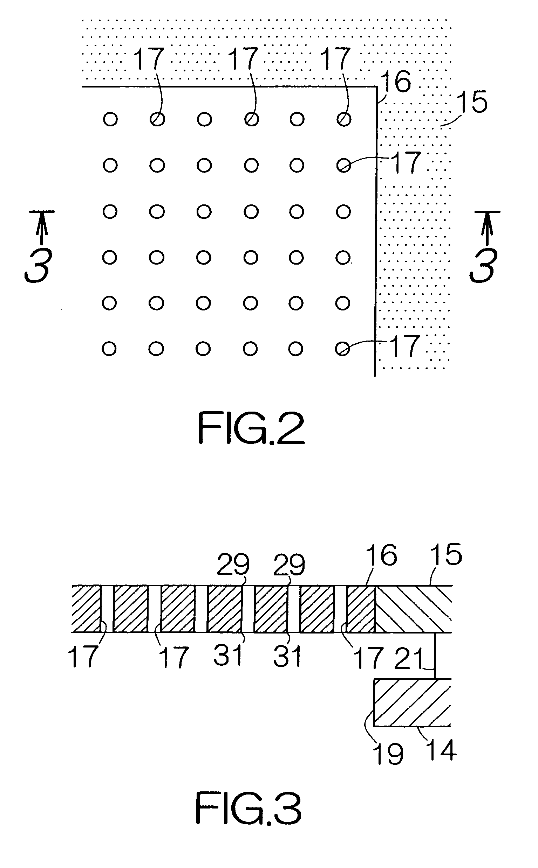

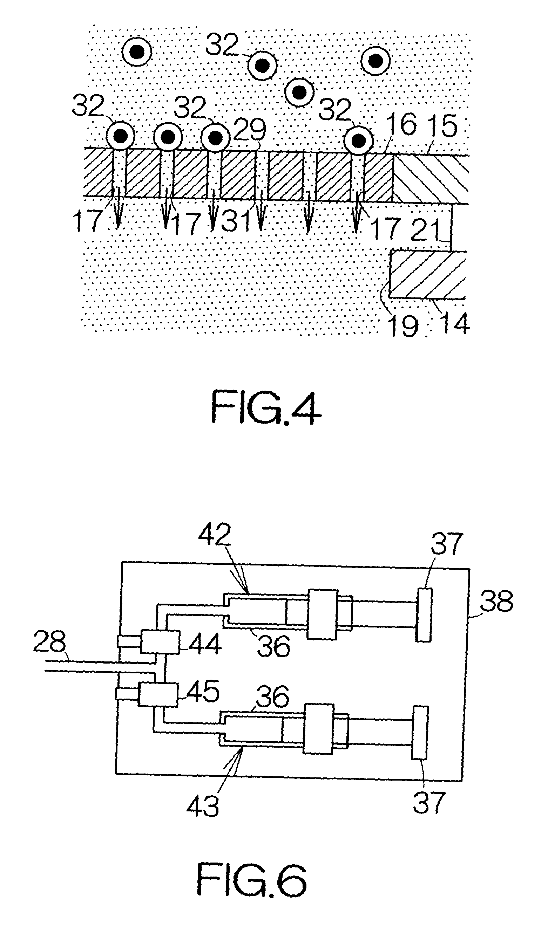

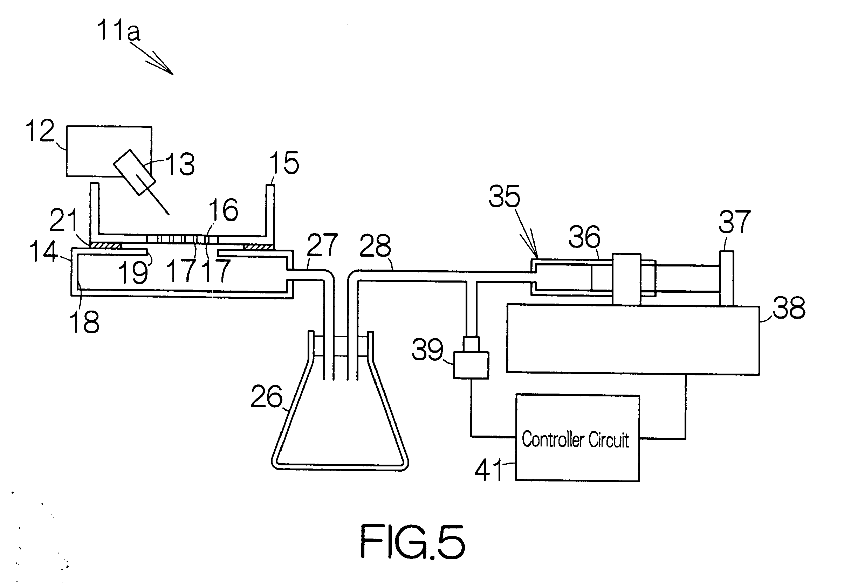

As shown in Fig. 2, the paths 17 are arranged at equal intervals in a matrix of eleven rows and eleven columns on the silicon chip 16. Each of the paths 17 may have a circular cross-section, for example. The diameter of the cross-section may be set smaller than at least the outside dimension of a cell. The cell may have a diameter in a range from 10µm to 20µm approximately, for example. In this case, the diameter of the cross-section of the path 17 is set in a range from 2µm to 3µm approximately. The space is set at 50µm approximately between the adjacent paths 17, for example. As shown in Fig. 3, each path 17 has a front opening 29 and a back opening 31. The front opening 29 is opened within the Petri dish 15. The back opening 31 is opened into the pressure chamber 18.

Next, a brief description will be made on the operation of the microinjection apparatus 11. First of all, the vacuum pump 22 starts working. In this case, the Petri dish 15 is not mounted on the work stage 14. The electropneumatic regulator 23 receives voltage of a predetermined value. The suction equal to or larger than 30 [kPa] is in this manner applied to the pressure chamber so as to generate negative pressure within the pressure chamber 18, for example. Air thus flows into the pressure chamber 18 from the external space through the opening 19 of the work stage 14. Culture medium or water drops remaining within the pressure chamber 18 and/or the first tube 27 drops onto the bottom of the liquid reservoir 26 from the opening of the first tube 27.

The Petri dish 15 is then mounted on the work stage 14. A culture medium is dropped onto the silicon chip 16 in the Petri dish 15. The culture medium fails to include any cells. The vacuum pump 22 then returns to the operation. Voltage of a predetermined level is supplied to the electropneumatic regulator 23. As described above, the suction equal to or larger than 30 [kPa] is applied to the pressure chamber 18 so as to generate negative pressure within the pressure chamber 18, for example. The vacuum pump 22 operates for five seconds. The paths 17 are in this manner filled with the culture medium.

Suspension is then dropped on the silicon chip 16 located in the Petri dish 15. The suspension includes cells dispersed in a culture medium. The cells are in this manner supplied to the silicon chip 16. The electropneumatic regulator 23 is supplied with voltage of a predetermined level suitable to the kind of cell within the suspension. In this case, the suction is set equal to 0.2[kPa] so as to generate negative pressure within the pressure chamber 18 and paths 17, for example. When the negative pressure is in this manner generated within the paths 17, the suspension in the Petri dish 15 flows into the paths 17.

Since the inside dimension of the paths 17 is set smaller than the outside dimension of cells, as shown in Fig. 4, the cells 32 in the suspension are caught at the front openings 29 of the paths 17. The electropneumatic regulator 23 serves to maintain the negative pressure equal to 0.2 [kPa] within the paths 17 all the time. The cells 32 are reliably kept immobilized on the front openings 29 of the paths 17. The capillary 13 is then inserted into each of the cells 32. Medicine within the capillary 13 is in this manner injected into the cells 32, for example.

The microinjection apparatus 11 enables establishment of a constant negative pressure within the paths 17 with the assistance of the electropneumatic regulator 23 all the time. Even if the suction of the vacuum pump 22 is forced to change in response to the pulsation of the vacuum pump 22, the negative pressure can be adjusted at a suitable level within the paths 17. Even if the negative pressure is forced to change in response to a change in the temperature of the paths 17, the pressure chamber 18 and the first and second tubes 27, 28, the negative pressure can be adjusted at a suitable level within the paths 17. Therefore, the cells 32 can reliably be kept immobilized at the front openings 29 of the paths 17.

In addition, the culture medium or the suspension drops onto the bottom of the liquid reservoir 26 from the opening of the first tube 27 after having flowed through the paths 17 based on the negative pressure. Since the second tube 28 is designed to have the opening at a location spaced from the bottom of the liquid reservoir 26 as described above, the culture medium or the suspension in the liquid reservoir 26 is prevented from flowing into the second tube 28. Only air is allowed to flow into the second tube 28. The electropneumatic regulator 23 and/or the vacuum pump 22 are reliably prevented from receiving the culture medium or suspension. The electropneumatic regulator 23 and the vacuum pump 22 are allowed to normally keep operating. This contributes to establishment of an accurate control on the negative pressure.

Moreover, the negative pressure is generated within the pressure chamber 17 and the first tube 27 prior to the attachment of the Petri dish 15 to the work stage 14. Culture medium or water drops remaining within the pressure chamber 18 and/or the first tube 27 is forced to drop onto the bottom of the liquid reservoir 26 from the opening of the first tube 27. As a result, the pressure chamber 18 and the first tube 27 can be prevented from suffering from generation of the surface tension at the boundary between air and the medium or between air and the drops. In addition, the paths 17 are completely filled with the culture medium prior to the capture of the cells 32 at the front openings 29 of the paths 17. The culture medium is prevented from mixture with air within the paths 17. Generation of the surface tension can reliably be avoided at the boundary between air and the culture medium within the paths 17. This contributes to establishment of an accurate control on the negative pressure within the paths 17.

Fig. 5 schematically illustrates the structure of a microinjection apparatus 11a including a specific example of a cell capturing apparatus according to a second embodiment of the present invention. A syringe 35 as a negative pressure generating device is incorporated in this microinjection apparatus 11a, in place of the aforementioned vacuum pump 22. The syringe 35 includes a cylinder 36 and a piston 37 located within the cylinder 36. The cylinder 36 is connected to the second tube 28. A driving mechanism 38 is connected to the piston 37. A syringe pump may be employed as the driving mechanism 38, for example. The driving mechanism 38 enables movement of the piston 37 relative to the cylinder 36. When the driving mechanism 38 drives the piston 37, the piston 37 is pulled back to expand the space within the cylinder chamber, the syringe 35 sucks air at a predetermined suction. The syringe 35 is in this manner capable of generating negative pressure in the pressure chamber 18 and the paths 17.

A pressure sensor 39 and a controller circuit 41 are incorporated within the microinjection apparatus 11a, in place of the aforementioned electropneumatic regulator 23, digital/analog converter 24 and computer 25. The pressure sensor 39 is connected to the second tube 28. The pressure sensor 39 is allowed to detect the negative pressure within the paths 17. The controller circuit 41 is connected to the pressure sensor 39 and the driving mechanism 38. The controller circuit 41 controls the driving mechanism 38 based on the negative pressure detected at the pressure sensor 39. The controller circuit 41 serves to control the amount of the movement of the piston 37. The negative pressure caused by the syringe 35 is allowed to enjoy a feedback control in this manner. Here, the pressure sensor 39 and the controller circuit 41 in combination serve as a negative pressure controlling device of embodiments of the present invention. Like reference numerals are attached to structure or components equivalent to those of the aforementioned first embodiment.

The controller circuit 41 supplies control signals to the driving mechanism 38. The driving mechanism 38 causes the piston 37 to move by a predetermined distance. The piston 37 is in this manner retreat from the cylinder 36. The pressure sensor 39 detects the level of the negative pressure generated within the paths 17. The detected level is notified to the controller circuit 41. The controller circuit 41 supplies the driving mechanism 38 with control signals based on the detected level of the negative pressure. The driving mechanism 38 correspondingly adjusts the amount of the movement of the piston 37. The negative pressure within the paths 17 is in this manner kept constant all the time.

The microinjection apparatus 11a likewise allows a stable capture of the cells 32 at the front openings 29 of the paths 17. In addition, if the syringe 35 is removably attached to the driving mechanism 38, the syringe 35 can easily be replaced. The syringe 35 is allowed to reliably enjoy sterilization and/or antibacterial treatment as compared with the aforementioned vacuum pump 22.

Otherwise, the microinjection apparatus 11a may include first and second syringes 42, 43 in place of the aforementioned syringe 35, as shown in Fig. 6. The first and second syringes 42, 43 may be arranged side by side in parallel. The second tube 28 may bifurcate at the end near the first and second syringes 42, 43. The aforementioned driving mechanism 38 is capable of individually driving the first and second syringes 42, 43. The driving mechanism 38 is also capable of simultaneously driving both the first and second syringes 42, 43.

A first valve 44 is incorporated in a branch of the second tube 28 between the bifurcated point and the first syringe 42. The first valve 44 serves to switch over connection between the first syringe 42 and the second tube 28 and connection between the first syringe 42 and the external space. A second valve 45 is likewise incorporated in a branch of the second tube 28 between the bifurcated point and the second syringe 43. The second valve 43 serves to switch over connection between the second syringe 43 and the second tube 28 and connection between the second syringe 43 and the external space.

The first syringe 42 is first connected to the second tube 28 through the first valve 44. In this case, the second syringe 43 is connected to the external space through the second valve 45. When the driving mechanism 38 drives the piston 37 in the first syringe 42 by a predetermined distance, the first syringe 42 generates negative pressure within the paths 17. For example, when the piston 37 reaches the rear end of the cylinder 36, the negative pressure cannot further be increased within the paths 17. Here, the second syringe 43 is connected to the second tube 28 through the second valve 45. The first syringe 42 is connected to the external space through the first valve 44.

The piston 37 is pulled back in the second syringe 43. The second syringe 43 in this manner generates negative pressure within the paths 17. The negative pressure is allowed to further increase within the paths 17. Since the first syringe 42 is connected to the external space, air can be discharged out of the cylinder 36. The piston 37 is pushed back so as to reach the front end position of the piston 37. When the second syringe 43 allows the piston 37 to reach the rear end position so as to maximize the space inside the cylinder 36, the first syringe 42 is allowed to take the place of the second syringe 43 based on the switchover of the first valve 44. The first and second syringes 42, 43 can thus be utilized by turns. The negative pressure can endlessly be increased within the paths 17. The microinjection apparatus 11a can thus be utilized in extensive purposes.

The microinjection apparatus 11, 11a, may allow utilization of a glass pipe such as a holding pipette, for example, in place of the aforementioned silicon chip 16. In this case, the holding pipette may be connected to: the first and second tubes 27, 28; the liquid reservoir 26; the negative pressure generating device such as the vacuum pump 22 and syringe 35; and the negative pressure controlling device such as the electropneumatic regulator 23 and the combination of the pressure sensor 39 and the controller circuit 41.

It will be appreciated by those skilled in the art that the invention has been described by way of example only, and that a variety of alternative approaches may be adopted without departing from the scope of the invention, as defined by the appended claims.

## Claims

1. A cell capturing apparatus comprising:
a path defining an opening smaller than a cell;
a negative pressure generating device connected to the path, said negative pressure generating device generating negative pressure within the path; and
a negative pressure controlling device connected to the path, said negative pressure controlling device adjusting the negative pressure within the path.

2. The cell capturing apparatus according to claim 1, further comprising:
a liquid reservoir defining a closed space;
a first piping member connected to the path, said first piping member having an opening within the liquid reservoir at a location spaced from a bottom of the liquid reservoir; and
a second piping member connected to the negative pressure generating device, said second piping member having an opening within the liquid reservoir at a location spaced from the bottom of the liquid reservoir.

3. A microinjection apparatus comprising:
a path defining an opening smaller than a cell;
a negative pressure generating device connected to the path, said negative pressure generating device generating negative pressure within the path;
a negative pressure controlling device connected to the path, said negative pressure controlling device adjusting the negative pressure within the path; and
an injector injecting a predetermined injectant into the cell.

4. A method of capturing a cell, comprising:
filling a path with solution, said path defining an opening smaller than the cell;
supplying the cell to the opening of the path; and
generating a predetermined negative pressure within the path so as to immobilize the cell at the opening of the path.
